# EUROPÄISCHE PATENTSCHRIFT

(11) **EP 3 221 647 B1**
(45) Veröffentlichungstag und Bekanntmachung des Hinweises auf die Patenterteilung: **19.12.2018**
(21) Anmeldenummer: 15798369.3
(22) Anmeldetag: 17.11.2015
(51) Int. Cl.: F24F 6/12, F24F 7/06, F24F 12/00

(54) **ANORDNUNG UMFASSEND EINEN INNENRAUM UND EINE LÜFTUNGSANLAGE UND VERFAHREN ZUM KLIMATISIEREN EINES INNENRAUMS MIT EINER SOLCHEN LÜFTUNGSANLAGE**
ARRANGEMENT OF AN ENCLOSURE AND A VENTILATION DEVICE AND METHOD FOR AIR CONDITIONING AN ENCLOSURE
ARRANGEMENT COMPRENANT UNE PIÈCE ET UN SYSTÈME DE VENTILATION ET MÉTHODE POUR CLIMATISER UNE PIÈCE

(30) Priorität: 18.11.2014 DE 202014105531 U; 23.02.2015 EP 15156137
(43) Veröffentlichungstag der Anmeldung: 27.09.2017
(73) Patentinhaber: Reichenbach, Albert, 58640 Iserlohn (DE)
(72) Erfinder: Reichenbach, Albert, 58640 Iserlohn (DE)
(74) Vertreter: Haverkamp, Jens
(86) Internationale Anmeldenummer: PCT/EP2015/076764
(87) Internationale Veröffentlichungsnummer: WO 2016/079088

(56) Entgegenhaltungen:
- WO-A1-99/06774

## Beschreibung

Die Erfindung betrifft eine Anordnung umfassend einen Innenraum und eine Lüftungsanlage, welche Lüftungsanlage ein Förderaggregat zum Fördern eines Luftstroms umfasst, welches Förderaggregat zumindest einen in dem Innenraum angeordneten Luftaustrittskanal mit einem Luftstrom beaufschlagt, wobei Teil der Lüftungsanlage eine Befeuchtungseinrichtung zum Zuführen von Luftfeuchte in den Innenraum ist, welche Befeuchtungseinrichtung einen Ultraschallvernebler zum Erzeugen eines Aerosols aus einer Flüssigkeit mit Flüssigkeitströpfchen in einer Größe, damit diese von der sich in dem Innenraum einstellenden Luftströmung mitgeführt werden können, eine Aerosolzuleitung zum Zuführen von Aerosol an zumindest einen Aerosolaustrittskanal sowie zumindest einen Aerosolaustrittskanal umfasst, der in dem Innenraum dergestalt angeordnet ist, damit das austretende Aerosol von der Luftströmung erfassbar ist. Beschrieben ist des Weiteren ein Verfahren zum Klimatisieren eines Innenraums unter Verwendung wenigstens einer solchen Lüftungsanlage.

Lüftungsanlagen dieser Art werden zum Belüften beziehungsweise Klimatisieren von Innenräumen eingesetzt. Bei Innenräumen der in Rede stehenden Art kann es sich um beliebige, prinzipiell geschlossene Räume handeln, wobei unter diesen Begriff auch der Innenraum eines gesamten Gebäudes oder Gebäudeabschnittes zu subsumieren ist. Somit kann ein Innenraum im Rahmen dieser Ausführungen auch ein Produktionsraum sein, beispielsweise auch eine Strecke in einem mehrprozessigen Produktionsablauf. Diese Anlagen dienen dem Zweck, in den Innenraum Zuluft zuzuführen und Abluft abzuführen. Bei der Zuluft handelt es sich typischerweise um Frischluft oder um einen Luftstrom mit einem Frischluftanteil, letzteres wenn der Abluftstrom oder ein Teil desselben rezirkuliert wird. Eine solche Lüftungsanlage umfasst im Innenraum einen Zuluftaustritt, der gemäß einer vorbekannten Ausgestaltung durch ein oder mehrere Luftaustrittsrohre ausgeführt ist. Um einen möglichst gleichmäßigen Luftaustritt über einen bestimmten Innenraumbereich zu erhalten, werden als Luftaustritt oftmals poröse Kanäle, beispielsweise Textilrohre eingesetzt, aus denen der Zuluftstrom austritt. Durch das Zuführen der Zuluft wird in dem Zuluftaustrittsbereich im Innenraum ein geringer Überdruck erzeugt.

Der Lüftungsanlage zugehörig ist zumindest eine Abluftöffnung. Diese ist in Bezug auf ihre freie durchströmbare Querschnittsfläche einstellbar. Auf Grund des durch die Zuluftzufuhr in dem Innenraum gebildeten Überdruckes stellt sich eine Luftströmung von dem zumindest einen Zuluftaustritt in Richtung zu der zumindest einen Abluftöffnung ein. Aus diesem Grunde sind der Zuluftzutritt und die Abluftöffnung voneinander beabstandet. In aller Regel wird man eine solche Lüftungsanlage im Innenraum derart anordnen, dass sich der Zuluftaustritt in Bezug auf die Höhe des Innenraumes auf einem tieferen Niveau befindet als die zumindest eine Abluftöffnung. Sodann kann über die gesamte gewünschte Grundfläche des Innenraumes Zuluft zugeführt und die Abluft auch unter Ausnutzung des sich ausbildenden Kamineffektes durch die zumindest eine Abluftöffnung abgeführt werden.

Mit einer solchermaßen konzipierten Lüftungsanlage wird nicht nur Frischluft in den Innenraum zugeführt, sondern dieser wird zugleich durch die sich einstellende Luftströmung sauber gehalten. Staub oder andere Schwebstoffe werden mit dem Abluftstrom weggeführt. Aus diesem Grunde werden derartige Lüftungsanlagen vielfach auch in Lebensmittel herstellenden Betrieben eingesetzt, beispielsweise Bäckereien, insbesondere Großbäckereien. In Abhängigkeit von der mit einem solchen Abluftstrom abgeführten Schwebstoffe können ein oder mehrere Filter vorgesehen sein, durch die der Abluftstrom tritt, bevor er, falls nicht vollständig rezirkuliert, über die Abluftöffnung an die Umgebung abgegeben wird.

Die Lüftungsanlage kann über ein Wärmerückgewinnungsaggregat - einen so genannten Rekuperator - verfügen, der aus dem Abluftstrom Wärme entnimmt und an den Zuluftstrom abgibt. Auf diese Weise kann in dem Innenraum generierte Prozesswärme, etwa durch den Betrieb Wärme abgebender Geräte genutzt werden, um der Zuluft Wärme zuzuführen. Mitunter verfügt eine solche Lüftungsanlage zusätzlich auch über eine Heizeinrichtung zum autarken Erwärmen der zuzuführenden Zuluft.

Lüftungsanlagen dieser Art müssen mitunter durch eine Klimaanlage ergänzt werden, wenn die Temperaturen im Innenraum entweder durch entstehende Prozesswärme oder durch Umgebungswärme über eine tolerablen Temperaturschwelle ansteigen können.

Zusätzlich kann es erforderlich sein, die relative Luftfeuchte in dem Innenraum auf einem bestimmten Niveau zu belassen oder eine solche einzustellen. In einem solchen Fall wird der Lüftungsanlage in eine Luftbefeuchtungsanlage zugeordnet sein. Die in Dampfform in den Innenraum eingebrachte Feuchtigkeit kann allerdings eine Kondensatbildung zur Folge haben, was vor allem bei einem Einsatz einer solchen Lüftungsanlage in einem Lebensmittel herstellenden oder verarbeitenden Betrieb nicht gewünscht wird.

Auch wenn derartige Lüftungs- bzw. -klimatisierungsanlagen den gewünschten Zwecken genügen, erweisen diese sich jedoch mitunter als aufwendig und kostenträchtig im Betrieb.

WO 99/06774 offenbart ein Verneblungssystem als klimatisierte Kühleinrichtung. Dieses vorbekannte System umfasst ein Rohr oder einen Schlauch, aus dem darin eingebrachte Luft unter Druck herausgedrückt wird, so dass auf diese Weise ein Luftstrom generiert wird. Über die Länge eines solchen Luftaustrittschlauches verteilt befinden sich mit Abstand zueinander eine Reihe von Dispersionsöffnungen. Diese sind als Düsen ausgelegt. Angeschlossen sind diese Düsen an eine Flüssigkeitszuführleitung, über die Mittels einer Pumpe an den Düsen zu zerstäubende Flüssigkeit zugeführt wird. Auf Grund des Luftstromes, in dem diese Düsen angeordnet sind, wird die in den Düsen zerstäubte Flüssigkeit mit dem Luftstrom mitgeführt. Durch den Flüssigkeitsnebel, der in warmer Umgebung verdampft, wird der Umgebung Wärme entzogen, was für den mit diesem Verneblungssystem gewünschten Kühleffekt verantwortlich ist.

Eine Anordnung mit den Merkmalen des Oberbegriffs des Anspruchs 1 ist aus FR 2 847 968 A1 bekannt. Diese vorbekannte Anordnung ist eine Einrichtung zur Luftfeuchtigkeitskontrolle von Weinfässern. Prinzipiell ist diese Lüftungsanlage aufgebaut, wie die aus WO 99/06774 A1 bekannte.

Ausgehend von diesem diskutierten Stand der Technik liegt der Erfindung daher die Aufgabe zu Grunde, eine eingangs genannte, gattungsgemäße Anlage für die Zuführung von Frischluft in einen Innenraum weiterzubilden und durch Befeuchtung dieser Frischluft Zusatzeffekte herbeizuführen, wie beispielsweise eine Kühlung.

Gelöst wird diese Aufgabe erfindungsgemäß durch eine eingangs genannte gattungsgemäße Anordnung, bei der das Förderaggregat zur Förderung eines Zuluftstroms in den Innenraum vorgesehen ist und dieses Förderaggregat auch den Ultraschallvernebler umfasst und bei der der Innenraum zumindest eine hinsichtlich ihrer Öffnungsweite einstellbare Abluftöffnung aufweist, durch die Abluft aus dem Innenraum austreten kann, wobei der zumindest eine Luftaustrittskanal mit Abstand zu der zumindest einen Abluftöffnung angeordnet ist, bei der sich bei einem Betrieb der Lüftungsanlage in dem Innenraum eine Luftströmung von dem zumindest einen Luftaustrittskanal zu der zumindest einen Abluftöffnung einstellt, wobei das austretende Aerosol von der Luftströmung erfasst wird der zumindest eine Aerosolaustrittskanal als Aerosolaustrittsrohr ausgeführt ist und jedes Aerosolaustrittsrohr in Bezug auf seine Erstreckung parallel und in funktionaler Nachbarschaft zu einem Luftaustrittskanal angeordnet ist.

Gelöst wird die Aufgabe auch durch ein Verfahren zum Klimatisieren eines Innenraums unter Verwendung wenigstens einer solchen Lüftungsanlage, bei dem durch wenigstens einen Luftaustrittskanal ein Zuluftstrom in das Gebäude mit einem dem an dieser Stelle ohne Zuluftstrom herrschenden Druck höheren Druck eingebracht wird, welcher Zuluftstrom durch das Gebäude oder einen Teil desselben zu wenigstens einer Abluftöffnung geleitet wird, und dass dem Zuluftstrom nach Austritt aus einem Luftaustrittskanal ein Aerosol mit Flüssigkeitstropfen in einer Größe beigemengt wird, damit diese von der sich in dem Gebäude einstellenden Luftströmung mitgeführt werden.

Bei der erfindungsgemäßen Anordnung mit ihrer Lüftungsanlage - gleiches gilt für das beanspruchte Verfahren - wird Luftfeuchte in Form eines Aerosols in den Innenraum eingebracht. Aus diesem Grunde umfasst diese Lüftungsanlage einen Nebelgenerator zum Erzeugen des Aerosols aus einer Flüssigkeit. Bei dem Generator kann es sich beispielsweise um einen Ultraschallvernebler handeln. Ein solcher Ultraschallvernebler erzeugt mittels hochfrequenter mechanischer Schwingungen die Flüssigkeitströpfchen in der gewünschten Größenordnung aus einem Flüssigkeitsfilm. Eingesetzt werden können hierzu beispielsweise piezokeramische Elemente, die elektrische Schwingungen in die notwendigen mechanischen Schwingungen umwandeln. Die Tröpfchengröße des erzeugten Aerosols ist hinreichend klein, damit diese von einem Luftstrom als Schwebfracht getragen werden können. Aus diesem Grunde ist der in dem Innenraum angeordnete Aerosolaustritt in Bezug auf den Zuluftaustritt, aus dem die Zuluft austritt, dergestalt angeordnet, dass das austretende Aerosol von der sich bei einem Betrieb der Lüftungsanlage in dem Innenraum einstellenden Luftströmung erfasst wird. Zusätzlich ist bei dieser Lüftungsanlage vorgesehen, dass die Tröpfchengröße des Aerosols eingestellt wird beziehungsweise eingestellt ist, dass diese von der sich in dem Innenraum einstellenden Luftströmung mitgeführt werden können, mithin durch diesen in Schwebe gehalten werden. Dieses Zusammenwirken zwischen der Tröpfchengröße und der Anordnung des Aerosolaustritts in Bezug auf den Zuluftaustritt im Innenraum trägt Sorge dafür, dass eine Kondensatbildung vermieden ist. Vor allem kondensatssensible Oberflächen werden durch Zuführen und in Schwebehalten der Luftfeuchte in der beschriebenen Art und Weise, wenn überhaupt, nur sehr gering belastet. Dieses wirkt sich kostenreduzierend auf die ansonsten notwendigen Maßnahmen zum Reinhalten derartiger Oberflächen aus. Vorteilhaft ist des Weiteren, dass die Zuführung der Luftfeuchte als Aerosol in konditionierter Weise erfolgen kann. Dies bedeutet, dass die zugeführten Flüssigkeitströpfchen konditioniert sein können, also: beispielsweise biologische oder andere Zusätze enthalten können, auch Duftstoffe. Bei derartigen biologischen Zusätzen kann es sich beispielsweise um antimikrobielle und/oder antifungale Stoffe handeln. Vorzugsweise handelt es sich hierbei um micellierte Naturstoffe. Vorteilhaft ist die Zuführung von Raumfeuchte in Form von Aerosol vor allem auch in der Lebensmittelindustrie, da ein Verpacken in einer solchen Umgebung sich verlängernd auf das mittlere Haltbarkeitsdatum auswirken kann. Zudem wirkt ein Lebensmittel, welches in einer solchen Umgebung verpackt worden ist, für Kunden attraktiver.

Da sich die als Aerosol zugeführte Luftfeuchte nicht als Kondensat in dem Innenraum niederschlägt, werden Schwebstoffe durch die Flüssigkeitströpfchen gebunden und durch die Luftströmung aus dem reinzuhaltenden bzw. zu konditionierenden Innenraum oder Innenraumbereich weggeführt.

Mit dem vorgeschriebenen Konzept kann nicht nur auf Feuchtigkeitsänderungen im Innenraum rasch und gezielt reagiert werden, sondern mit diesem Konzept kann die Luftfeuchtigkeit durch entsprechende Steuerung der Aerosolzuführung sehr konstant gehalten werden/oder an sich ändernde Umgebungseinflüsse angepasst werden in dem gewünschten Maße von 50 bis 55 Prozent gehalten werden. Die Luftfeuchte im Innenraum kann, wenn gewünscht, auch auf einen höheren Wert eingestellt werden.

Zur Innenraumluftbefeuchtung wird man typischerweise steriles Wasser für die Aerosolerzeugung verwenden. Damit die Tröpfchen von dem sich im Innenraum bei einem Betrieb der Lüftungsanlage einstellenden Luftstrom mitgeführt werden können, liegen diese in der Größenordnung typischerweise zwischen 1 bis 5 µm, können jedoch, wenn gewünscht, auch Größen von etwa 20 µm erreichen.

Um eine über den zu belüftenden Innenraumbereich gleichmäßige Belüftung zu erreichen, ist in einem Ausführungsbeispiel einer Lüftungsanlage vorgesehen, dass der zumindest eine Luftaustrittskanal als Textilrohr ausgeführt ist. Typischerweise wird man in einer bestimmten Höhe des Innenraums ein oder mehrere derartige Textilrohre anordnen. Diese können unterschiedliche Querschnittsgeometrien und auch unterschiedliche Geometrien in ihrer Längserstreckung aufweisen. Die Geometrien der Längserstreckung wird man an die räumlichen Gegebenheiten anpassen wollen. Durch ein solches Textilrohr tritt die Zuluft durch die Textilwandung hindurch. Erreicht wird hierdurch ein flächiger Austritt der Zuluft. Das Austreten der Zuluft erfolgt mit einem gewissen Überdruck mit dem Ergebnis, dass sich in dem zu belüftenden Teil des Innenraumes, in dem der Luftaustritt erfolgt, ein gewisser Überdruck aufbaut und bei einem kontinuierlichen Betrieb der Lüftungsanlage herrscht. Zugeführt wird die Zuluft an das oder die Luftaustrittskanäle über Zuleitungsrohre. Typischerweise befindet sich das Förderaggregat zum Fördern der Zuluft außerhalb des Innenraumes, zumeist auf dem Gebäudedach.

Bei einer Ausgestaltung des Zuluftaustrittes durch Verwenden ein oder mehrerer Textilrohre wird man den Aerosolaustritt ebenfalls als Kanal, etwa als Rohr ausführen, und zwar dergestalt, dass sich ein solches Aerosolaustrittsrohr parallel zu dem Zuluftaustrittskanal erstreckt und in geringem Abstand zu diesem verläuft. Vorteilhafterweise wird man den Aerosolaustritt etwas oberhalb des Zuluftaustrittes anordnen, damit das austretende Aerosol in die sich einstellende Luftströmung im Innenraum, beginnend an dem Zuluftaustritt schwerkraftbedingt quasi in diese hineinfällt und sodann von der Luftströmung erfasst und mitgeführt wird. Insofern ist von Vorteil, dass die Zuluftströmung nicht aus einzelnen Düsen oder Öffnungen, sondern durch die gesamte oder einen nennenswerten Anteil der Mantelfläche eines solchen Textilrohres austritt. Dieser austretende Zuluftstrom, der aufgrund des vorbeschriebenen Austritts bereits ein nennenswertes Volumen hat, nimmt das aus dem Aerosolaustritt austretende Aerosol als Schwebfracht auf und führt dieses mit.

Vorteilhaft ist zum Betrieb einer solchen Lüftungsanlage, wenn sich in dem zu belüftenden Innenraum ein gewisser Kamineffekt einstellt. Dieser stellt sich beispielsweise dann ein, wenn Prozesswärme im Innenraum entsteht. Dieses wird erreicht, indem in Bezug auf die Höhe des Innenraums der Zuluftaustritt auf einem tieferen Niveau angeordnet ist als die zumindest eine Abluftöffnung. Auch eine Kombination dieser Maßnahmen ist möglich.

Es versteht sich, dass sich eine solche Lüftungsanlage über mehrere Etagen im Innenraum erstrecken kann, die vorzugsweise untereinander verbunden sein können.

Die zum Betrieb der Lüftungsanlage notwendigen Komponenten sind vorzugsweise sämtlich dem Förderaggregat zugeordnet. Dieses umfasst die zum Fördern des Zuluftstroms benötigte Luftförderpumpe und den Nebelgenerator. Der Nebelgenerator ist an eine Flüssigkeitsversorgung angeschlossen. In aller Regel werden zusätzliche Luftfilter verwendet, um dem Innenraum gefilterte Zuluft zuzuführen. Ist eine Rückgewinnung von Wärme aus dem Abluftstrom vorgesehen, wird man das hierfür benötigte, zumindest eine Wärmerückgewinnungsaggregat ebenfalls dem Förderaggregat zuordnen, sodass sämtliche Elemente in einer Baueinheit zusammen angeordnet sind.

Ein solches, bezüglich der notwendigen Elemente in einem Gehäuse zusammengefasstes Förderaggregat ist gemäß einer bevorzugten Ausgestaltung auf Stützen, typischerweise auf dem Dach eines Gebäudes aufgestellt. Dabei sind die Komponenten eines solchen Förderaggregates in dem Gehäuse angeordnet, damit diese bei Bedarf, insbesondere die Filter, herausgenommen, gereinigt und/oder ausgetauscht werden können. Eine Anordnung des Förderaggregates mit seinem Gehäuse auf Stützen ist vor allem dann vorteilhaft, wenn eine Innenreinigung der einzelnen Aggregate und/oder des Gehäuses flüssigkeitsbasiert vorgesehen ist. Dann wird man das Gehäuse in einer Wanne vorsehen oder bodenseitig eine Flüssigkeitsaufnahmerinne umlaufend um den Boden des Gehäuses vorsehen. Beides dient zur Aufnahme von Reinigungsflüssigkeit. Damit diese nicht wahllos aus der Flüssigkeitsaufnahmerinne oder der Wanne ausläuft, sind ein oder mehrere Abflussöffnungen vorgesehen. Aus diesen kann das Reinigungswasser austreten und, sollte sich das Förderaggregat auf dem Dach eines Gebäudes befinden, der Dachentwässerung zugeführt werden. Möglich ist es bei einer solchen Ausgestaltung auch, an die zumindest eine Abflussöffnung eine Leitung, etwa ein Rohr oder einen Schlauch anzuschließen, durch das beziehungsweise die das Reinigungsabwasser entsorgt wird, beispielsweise einer Reinigungswasseraufbereitung zugeführt wird.

Gemäß einem bevorzugtem Ausführungsbeispiel ist vorgesehen, die Lüftungsanlage in eine in einem Gebäude befindliche Produktionsstätte zu integrieren.

Eine Zuführung eines beispielsweise mit biologischen Zusätzen ausgerüstetem Aerosols kann vorgenommen werden, um beispielsweise in zeitlichen Abständen einem oder auch mehreren Arbeitsbereichen Fungizide zuzuführen, sollte dieses erforderlich oder sinnvoll sein. Zweckmäßig ist eine solche Maßnahme beispielsweise bei einem Arbeitsbereich, bei dem es sich um eine Reinigungs- oder Waschstation handelt, in der beispielsweise Gebinde gereinigt oder gewaschen werden, welche Gebinde anschließend als Transportbehälter für Lebensmittel dienen. Auf diese Weise kann unter Ausnutzung der besonderen Verbreitung des zugeführten Aerosols innerhalb eines Arbeitsbereiches wirksam einer Schimmelbildung etwa an den Wänden entgegengewirkt werden. Eine solche Arbeitsbereichsbehandlung wird man bevorzugt vornehmen, wenn der Betrieb in diesem Arbeitsbereich ruht.

Zur Konditionierung können auch Duftstoffe zugeführt werden. Dieses bietet sich beispielsweise in Verkaufsräumen zur Unterstützung des Einkaufverhaltens an.

Besonders vorteilhaft wirkt sich die Luftfeuchtezuführung in Form von Aerosol beispielsweise bei in einem Arbeitsbereich einer Bäckerei als beispielhafte Produktionsstätte befindlichen Teiglinge aus, auf denen sich das Aerosol in einem dünnen Film legt. Verbessert wird hierdurch nicht nur die Qualität des Teiglings und das Backergebnis, sondern, wie vorstehend bereits angedeutet, auch das mittlere Haltbarkeitsdatum, innerhalb dessen der Teigling gebacken werden soll.

Der Konzeption der Lüftungsanlage und der dieser zugeordneten Innenraumbefeuchtungseinrichtung folgend, wird man die Arbeitsbereiche in einer solchen Produktionsstätte einrichten. Diejenigen Arbeitsbereiche, in denen ein möglichst konstanter Luftdruck und eine besondere Sauberkeit herrschen soll, befinden sich unmittelbar im Bereich der Luftaustrittskanäle. Aufgrund der sich einstellenden Luftströmung in dem Gebäude wird man diejenigen Arbeitsbereiche, die in Richtung der Luftströmung zu dem oder den Abluftöffnungen hin angeordnet sind, für Arbeitsvorgänge nutzen, in denen nicht so hohe Anforderungen an Luftdruck und Sauberkeit gestellt sind, als dass diese unmittelbar im Bereich eines oder mehrerer Luftaustrittskanäle sich befinden müssten.

Für den Fall, dass in unterschiedlichen Arbeitsbereichen eine von anderen Arbeitsbereichen unabhängige Zuluftzufuhr über das oder die darin befindlichen Luftaustrittskanäle und eine Aerosolzuführung vorgesehen ist, ist vorzugsweise jedem solchen Arbeitsbereich ein eigenes Förderaggregat, umfassend eine Pumpe für die Zuluftzufuhr sowie einen Nebelgenerator zum Erzeugen des Aerosols, zugeordnet. Auch wenn auf diese Weise für die gesamte Lüftung und Klimatisierung des Gebäudes etliche Förderaggregate benötigt werden, so erlaubt eine solche Auslegung der Produktionsstätte eine besonders unabhängige Regulierung der Raumbedingungen in unterschiedlichen Arbeitsbereichen. Zudem kann in einem solchen Fall auf handelsübliche Aggregate zurückgegriffen werden, so dass die Gesamtkosten einer solchen Anlage nicht aus dem Rahmen fallen.

Die geschickte Auslegung des Gebäudes der Produktionsstätte zum Ermöglichen einer oder mehrerer Luftströmungen und das Nutzen der Prozesswärme einzelner Produktionsmaschinen als Luftströmungsmotor erlaubt einen Betrieb der Lüftungs- und Klimaanlage einer solchen Produktionsstätte mit nur sehr geringen Kosten, verglichen mit den Kosten, die für die Errichtung und den Betrieb herkömmlicher Lüftungs- und Klimatisierungsanlagen benötigt werden.

Damit die Luftströmung sich innerhalb des Gebäudes der Produktionsstätte ausbilden kann, ist dieses nach Art eines Niedrigenergiehauses hinreichend dicht. Bei Türen und Toren zwischen zwei unterschiedlichen Druckbereichen in dem Gebäude oder auch nach außen hin wird man typischerweise eine Schleuse zwischen den Bereichen unterschiedlichen Druckes vorsehen.

Bevorzugt weist eine solche Produktionsstätte in ihrem Gebäude mehrere Arbeitsbereiche auf, die typischerweise durch Wände voneinander getrennt sind. Dieses ist jedoch nicht unbedingt erforderlich und richtet sich nach den in einem Arbeitsbereich zu verrichtenden Arbeiten. In denjenigen Arbeitsbereichen, in denen erhöhte Anforderungen an einen gleichbleibenden Luftdruck und an Sauberkeit gestellt sind, werden eine oder mehrere Luftaustrittskanäle, typischerweise zusammen mit entsprechenden Aerosolaustrittskanälen angeordnet. In den einzelnen Arbeitsbereichen sind vorzugsweise die Zuluftzufuhr und die Aerosolzufuhr von anderen Arbeitsbereichen mit einer Luft- und Aerosolzufuhr unabhängig einstellbar. Auf diese Weise kann im Bezug auf den in dem Arbeitsbereich gewünschten Luftdruck und die in einem Arbeitsbereich gewünschte Luftfeuchte unabhängig von den Anforderungen in anderen Arbeitsbereichen eingestellt werden. Aufgrund des Lüftungskonzeptes bei einer solchen Produktionsstätte besteht eine Luftströmungswegsamkeit von jedem Arbeitsbereich bis zu der oder den Abluftöffnungen des Gebäudes. Diese befinden sich vorzugsweise am Ende eines Abluftströmungsschachtes, über den die Abluft der oder den Abluftklappen zugeführt wird. Insofern stellt ein solcher Abluftströmungsschacht einen Sammler für die durch den Überdruck in den oder in einzelnen Arbeitsbereichen erzeugte Luftströmung dar. Um dieses zu erreichen, sind Arbeitsbereiche, die durch Wände eingefasst sind, luftströmungstechnisch an einen solchen Abluftstromschacht angeschlossen. Dieses kann beispielsweise realisiert werden, indem die Arbeitsbereiche in einer Produktionsebene in ihren Wänden Strömungsdurchbrechungen aufweisen. Es versteht sich, dass diese hinreichend groß sein müssen, damit sich aufgrund des in aller Regel nur geringen Druckunterschiedes zwischen dem Druck im Bereich der Luftaustrittskanäle und der oder den Abluftöffnungen die gewünschte Luftströmung ausbilden kann. Möglich ist auch eine Luftströmung, bei der diese den jeweiligen Arbeitsbereich nach oben hin verlässt. Bei einer Produktionsstätte mit mehreren Produktionsebenen kann dieses beispielsweise durch Deckendurchbrechungen nach Art von Lufträumen erreicht werden. Es versteht sich, dass auch eine Kombination dieser Maßnahmen zum Realisieren der gewünschten Strömungswegsamkeit genutzt werden können.

Zwischen den einzelnen Arbeitsbereichen, die sich durchaus, wenn in einer Ebene befindlich, durch verschiedene Raumhöhen unterscheiden können, sind diese vorzugsweise durch Strömungsbarrieren voneinander getrennt. Dieses dient dem Ziel, den Luftstromübergang aus dem einen Arbeitsbereich in den anderen Arbeitsbereich zu kontrollieren. Verwirklichen lässt sich dieses beispielsweise von der Decke herabhängende Schotten.

Besonders effektiv und vor allem energieeffektiv arbeitet eine solche Entlüftungs- und Klimatisierungsanlage, wenn in dem Gehäuse der Produktionsstätte mehrere Produktionsebenen vorgesehen sind und für den Produktionsprozess ein oder mehrere Prozesswärme abgebende Produktionsmaschinen benötigt werden. Im Falle einer Bäckerei handelt es sich bei den Prozesswärme abgebenden Produktionsmaschinen beispielsweise um die verwendeten Öfen, vor allem wenn als Backöfen Durchlauföfen eingesetzt werden. Für die Luftstromgenerierung bzw. für die Unterstützung des sich allein aufgrund der Lüftungsanlage einstellenden Luftströmung wird man bei einer solchen Produktionsstätte diese Produktionsmaschinen dort aufstellen, wo die Luftströmung oder zumindest ein Teil derselben vorbei strömt. Die durch die abgegebene Prozesswärme bedingte Luftkonvektion unterstützt die in dem Gebäude vorgesehene Luftströmung. Aus diesem Grunde wird man eine solche Prozesswärme abgebende Produktionsmaschine in einem Gebäude mit mehreren Produktionsebenen in einer oberen Produktionsebene nahe oder sogar im Bereich eines Abluftstromschachtes anordnen. Der sich bei einer mit einer solchen Lüftungsanlage ausgerüsteten Produktionsstätte einstellende Kamineffekt wird durch eine solche Maßnahme in nicht unerheblichem Maße verstärkt. Da als Motor der Luftströmung sodann auch eine solche Prozesswärme abgebende Produktionsmaschine zur Verfügung steht, kann, ohne befürchten zu müssen, dass eine Luftströmung zwischen dem bzw. den Luftaustrittskanälen und der bzw. den Abluftöffnungen zum Stillstand kommt, der Zuluftstrom hinsichtlich seines Druckes in einer größeren Bandbreite eingestellt werden. Mithin stellt sich eine Luftströmung auch mit niedrigeren Drücken bzw. Druckdifferenzen zwischen Zuluft und Abluftöffnung ein, bei denen ohne eine Prozesswärme abgebende Produktionsmaschine als Luftstrommotor sich eine den gewünschten Zwecken genügende Luftströmung zwischen dem oder den Luftaustrittskanälen und dem oder den Abluftöffnungen nicht einstellen würde.

Typischerweise wird man in dem oder in einzelnen Arbeitsbereichen in einer bestimmten Höhe ein oder mehrere derartige Textilrohre als Luftaustrittsrohre anordnen. Diese können unterschiedlichen Querschnittsgeometrien und auch unterschiedliche Geometrien in ihrer Längserstreckung aufweisen. Die Geometrien der Längserstreckung wird man an die räumlichen Gegebenheiten anpassen wollen. Durch ein solches Textilrohr tritt die Zuluft durch die Textilwandung hindurch. Dieses erfolgt mit einem gewissen Überdruck mit dem Ergebnis, dass in den zu belüftenden Bereich, in dem der Luftaustritt erfolgt, ein gewisser Überdruck herrscht. Zugeführt wird die Zuluft an das oder die Luftaustrittskanäle über Zuleitungsrohre. Typischerweise befindet sich das hierzu notwendige Förderaggregat außerhalb des Gebäudes, zumeist auf dem Gebäudedach.

Bei einer Ausgestaltung des Zuluftaustrittes durch Verwenden ein oder mehrerer Textilrohre wird man den Aerosolaustritt ebenfalls als Kanal, etwa als Rohr ausführen, und zwar dergestalt, dass sich ein solches Aerosolaustrittsrohr parallel zu dem Zuluftaustrittskanal erstreckt und in geringem Abstand zu diesem verläuft. Vorteilhafterweise wird man den Aerosolaustritt etwas oberhalb des Zuluftaustrittes anordnen, damit das austretende Aerosol in die sich einstellende Luftströmung, beginnend an dem Zuluftaustritt, schwerkraftbedingt quasi in diese hineinfällt und sodann von der Luftströmung erfasst und nach Art von Schwebfracht mitgeführt wird.

Eine solche Produktionsstätte wird man vorzugsweise mit möglichst homogener und mit einem niedrigen Wärmedurchgangskoeffizienten versehener Oberfläche nach außen und daher prinzipiell unter Anwendung des so genannten Niedrigenergiehausstandards auslegen und anfallende Prozesswärme für eine Erwärmung der Zuluft, falls notwendig, und/oder für andere Wärme benötigende Produktionsprozesse nutzen. Die Auslegung einer Produktionsstätte mit einem solchen Standard ist somit dergestalt vorgesehen, dass die geforderten energietechnischen Anforderungsniveaus in der Summe unterschritten werden. Gemäß einem bevorzugten Ausführungsbeispiel wird der oder es werden die sich einstellenden Luftströmungen im Bezug auf ihre jeweilige Temperatur überwacht. Zudem wird denjenigen Arbeitsbereichen, in denen ein möglichst konstanter Luftdruck herrschen soll, der Luftdruck überwacht. Zum Zwecke einer Wärmebilanzierung wird ebenfalls die Abluftstrommenge und deren Temperatur überwacht. Die Daten der hierfür eingesetzten Sensoren beaufschlagen eine zentrale Steuereinheit, die dann wiederum zum Nachregeln von Schwankungen in Bezug auf die gewünschte Temperatur, den gewünschten Luftfeuchtigkeitsgehalt und den gewünschten Luftdruck den oder die entsprechenden Aktoren entsprechend ansteuert.

Im Rahmen des Betriebs einer solchen Lüftungsanlage, vor allem in Bereichen mit einer größeren Anzahl an Personen, wie etwa in Schulungs-, Büro- oder Kantinenräumen kann mit Frischluftzufuhr gekühlt werden, um auf diese Weise den CO₂-Gehalt zu reduzieren, was einem zu raschen Ermüden entgegenwirkt.

Die Leitungen und insbesondere die Aerosolleitungen werden typischerweise regelmäßig entkeimt, beispielsweise mit Ozon.

Um einer Kondensatansammlung in der Aerosolleitung, sollte dieses tatsächlich eintreten, entgegenzuwirken, können diese Leitungen mit einem gewissen Gefälle verlegt werden.

Besonders effektiv lässt sich eine solche Produktionsstätte betreiben, wenn diese ausgelegt ist, eine gewisse Speicherkapazität für die Klimatisierungsvariablen Temperatur, Feuchte und Druck aufweist. Bezüglich der Raumluftfeuchtigkeit kann dieses durch Bereitstellen einer gewissen Speichermasse, etwa über die Wände erreicht werden. Bezüglich der Druckdichtigkeit kann dieses durch Schotte, Schleusen, oder dergleichen neben einer bereits beschriebenen Außenabdichtung verwirklicht werden. Als Temperaturpuffer können ebenfalls die Wände und auch in einem Arbeitsbereich befindliche Gegenstände dienen. Im Zusammenhang mit einer bezüglich dieser Klimatisierungsvariablen vorgesehenen Steuerung des oder der Arbeitsbereiche der Produktionsstätte wird man die Be- und Entlüftung kontrollieren.

Mit einer solchen Lüftungsanlage kann, sollte dieses erforderlich sein, Druckluftschwankungen in der Umgebung ausgeglichen werden. Zu Nutze macht man sich die in dem Innenraum sich einstellende Luftströmung, generiert durch die mit einem gewissen Überdruck zugeführte Zuluft. Insofern kann in einem gewisse Maße über den Druck, mit dem die Zuluft zugeführt wird, in dem gesamten Innenraum oder in Teilen desselben derartige, typischerweise witterungsbedingte Druckunterschiede in der Außenumgebung begegnet werden. Nachfolgend ist die Erfindung anhand von Ausführungsbeispielen unter Bezugnahme auf die beigefügten Figuren beschrieben. Es zeigen:
- **Fig. 1:**: in einem vertikalen Schnitt ein Gebäude mit einer Lüftungsanlage zum Erläutern des beanspruchten Konzeptes und
- **Fig. 2:**: einen vertikalen Schnitt durch ein Gebäude einer Produktionsstätte mit einer nach den Prinzipien der Lüftungsanlage der Figur 1 arbeitenden Lüftungs- und Klimatisierungsanlage.

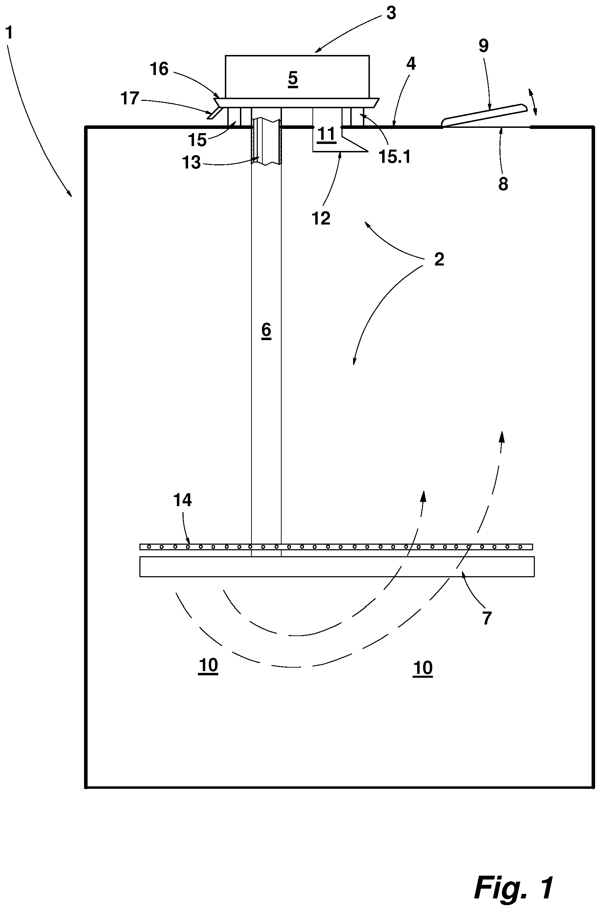

Zunächst wird das Lüftungs- und Klimatisierungsprinzip, wie dieses bei der beanspruchten Produktionsstätte eingesetzt wird, anhand der Figur 1 erläutert.

Figur 1 zeigt in einer stark schematisierten Darstellung einen vertikalen Schnitt durch ein Gebäude 1 mit einer Lüftungsanlage 2. Die Lüftungsanlage 2 umfasst ein Förderaggregat 3, welches auf dem Dach 4 des Gebäudes 1 aufgestellt ist. Die einzelnen Teile des Förderaggregats 3 sind in einem gemeinsamen Gehäuse 5 angeordnet und in der Figur 1 nicht näher dargestellt. Das Förderaggregat 3 umfasst eine Luftförderpumpe, durch die Zuluft in eine Zuluftleitung 6 gefördert wird. Das Förderaggregat 3 umfasst des Weiteren zumindest einen Filter, durch den die aus der Umgebung entnommene Zuluft vor Einbringen derselben in die Zuluftleitung 6 gefiltert wird. Durch die Zuluftleitung 6 ist das Förderaggregat 3 beziehungsweise die darin enthaltene Luftförderpumpe mit einem Luftaustrittskanal 7 verbunden. Bei dem Luftaustrittskanal 7 des dargestellten Ausführungsbeispiels handelt es sich um ein an sich bekanntes Textilrohr. Aus diesem wird die in die Zuluftleitung 6 geförderte Zuluft aufgrund der porösen Eigenschaften der Wandung heraus gedrückt.

Der Lüftungsanlage 1 ist zumindest eine Abluftöffnung 8 zugeordnet. Diese ist bei dem dargestellten Ausführungsbeispiel durch eine Abluftklappe 9 realisiert, die hinsichtlich ihrer Öffnungsweite und damit hinsichtlich ihrer freien durchströmbaren Querschnittsfläche einstellbar ist. Es versteht sich, dass die Abluftklappe 9 nur beispielhaft als Glied zum Einstellen der Öffnungsweite der Abluftöffnung 8 gezeigt ist. An dieser Stelle können auch Lamellen oder andere die Öffnungsweite der Abluftöffnung ändernde Stellglieder eingesetzt werden. Auch muss sich die Abluftöffnung 8 nicht, wie bei dem dargestellten Ausführungsbeispiel gezeigt, in dem Dach 4 des Gebäudes befinden, sondern kann ebenfalls an ein oder mehreren Seitenwänden desselben angeordnet sein. In Abhängigkeit von der Stellung der Abluftklappe 9 ist die Abluftöffnung 8 größer oder kleiner, sodass durch die Stellung der Abluftklappe 9 der an die Umgebung abgegebene Abluftstrom einrichtbar ist. Die Verstellbarkeit der Abluftklappe 9, die bei dem dargestellten Ausführungsbeispiel durch einen elektrischen Stellmotor bewirkt wird, ist durch den benachbart zu dieser in der Figur 1 eingetragenem Doppelpfeil kenntlich gemacht.

Das Förderaggregat 3 verfügt zudem über eine Rezirkulationswegsamkeit, über die Abluft durch das Förderaggregat 3 hingeführt werden kann. Eine solche Wegsamkeit kann ausgebildet sein, Abluft dem Zuluftstrom zuzuführen und/oder um Wärme aus dem Abluftstrudel eines Wärmetauscheraggregates zu entnehmen und dem Zuluftstrom zuzuführen. Letzteres ist vor allem dann zweckmäßig, wenn in dem Innenraum 10 des Gebäudes Prozesswärme entsteht und abzuführen ist. Dann kann diese genutzt werden, um beispielsweise in Wintermonaten kalte Zuluft aus der Umgebung zu erwärmen. Eine solche Wärmerückgewinnung wirkt sich positiv auf die Energiebilanz bei einem Betrieb der Lüftungsanlage und somit bezüglich der Betriebskosten des Gebäudes 1 aus. Zum Einleiten eines solchen rezirkulierenden Abluftstromes ist dem Förderaggregat 3 Abluftstutzen 11 zugeordnet, der mit seiner Öffnung 12 etwas unterhalb der Decke 4 des Gebäudes 1 angeordnet ist. In Abhängigkeit von der Stellung der Abluftklappe 9 kann der in den Abluftstutzen 11 eintretende Abluftstrom beziehungsweise Abluftstromanteil eingestellt werden.

Das Förderaggregat 3 umfasst des Weiteren einen Nebelgenerator zum Erzeugen eines Aerosols. Das Aerosol wird über eine Aerosolzuleitung 13 einem in den Innenraum 10 angeordneten Aerosolaustrittsrohr 14 zugeführt. Bei dem dargestellten Ausführungsbeispiel ist die Aerosolzuleitung 13 innerhalb der Zuluftleitung 16 angeordnet und in nicht dargestellten Art und Weise aus der Zuluftleitung 6 in Höhe des Aerosolsaustrittsrohrs 14 herausgeführt. Das Aerosolaustrittsrohr 14 verfügt über Öffnungen, aus denen das zugeführte Aerosol austritt. Das Aerosolaustrittsrohr 14 befindet sich in Bezug auf die Höhe des Innenraums 10 mit geringem Abstand oberhalb des als Textilrohr ausgeführten Luftaustrittskanals 7. Das austretende Aerosol fällt sodann schwerkraftbedingt an dem Luftaustrittskanal 7 vorbei in die sich zwischen Luftaustrittskanal 7 und der Abluftöffnung 8 und/oder dem Abluftstutzen 11 einstellende Luftströmung. Die durch den Nebelgenerator erzeugte Tröpfchengröße ist hinreichend klein, damit die Aerosoltröpfchen von dieser Luftströmung als Schwebfracht mitgeführt werden. Die sich einstellende Luftströmung ist in Figur 1 im Bereich des linken Teils des Luftaustrittskanals 7 schematisiert angedeutet. Durch diese schematisierte Darstellung der Luftströmung soll gezeigt werden, dass diese durch den zu belüftenden Innenraum 10 des Gebäudes 1 hindurchgeführt wird und dann nach oben in Richtung zur Abluftöffnung 8 aufsteigt. Insofern befindet sich das Luftaustrittskanals 7 in Bezug auf die Höhe des Gebäudes 1 auf einem deutlich tieferen Niveau als die Abluftöffnung 8.

Eine Zuführung der Luftfeuchte in Form von kleinsten Flüssigkeitströpfchen eines Aerosols hat nicht nur eine Zuführung von Luftfeuchte in den Innenraum 10 zur Folge. Vielmehr führt die Zuführung der Luftfeuchte in dieser Form zugleich zu einer adiabatischen Kühlung. In dem Innenraum 8 entstehende Prozesswärme wird somit nicht nur durch den Abluftstrom abgeführt, sondern zugleich gekühlt. Entsprechend gering ist der Aufwand, für den Fall, dass der Innenraum 10 zusätzlich klimatisiert, also: gekühlt, werden müsste.

Das in dem Gehäuse 5 befindliche Förderaggregat 3 ist auf dem Dach 4 des Gebäudes 1 auf Stützen 15, 15.1 aufgestellt. Das Gehäuse 5 ist in einer Flüssigkeitsauffangwanne 16 angeordnet. Die Wanne 16 verfügt über eine Ablauföffnung, an die ein Ablaufrohrstück 17 angeschlossen ist. Das Vorsehen der Wanne 16 und des Ablaufrohrstückes 17 dienen dem Zweck, Reinigungsflüssigkeit, die vor allem bei der Innenreinigung des Gehäuses 5 beziehungsweise darin vorhandener Aggregate anfällt, zielgerichtet der ohnehin vorhandenen Dachentwässerung zuzuführen.

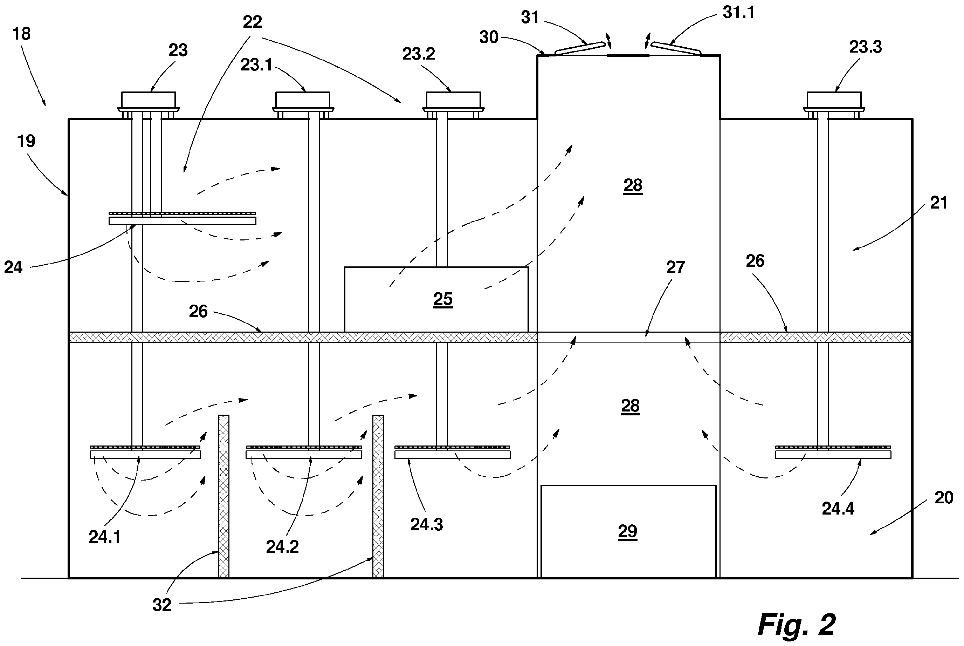

Figur 2 zeigt eine Produktionsstätte 18 mit einem mehrgeschossigen Gebäude 19, wobei bei dem dargestellten Ausführungsbeispiel zwei Produktionsetagen 20, 21 vorgesehen sind. Bei der Produktionsstätte 18 handelt es sich um eine Bäckerei mit integrierter Konditorei. Neben dem Gebäude 19 verfügt die Produktionsstätte 18 über eine Lüftungsanlage 22, die aufgebaut ist, wie die Lüftungsanlage 2 des Gebäudes 1. Die Lüftungsanlage 22 umfasst mehrere Förderaggregate 23, 23.1, 23.2, 23.3, die über Zuluftleitungen jeweils ein Luftaustrittsrohr mit zumindest einem diesem zugeordneten Aerosolaustrittsrohr mit Zuluft und Aerosol beaufschlagen. Diese Einheiten der Lüftungsanlage 22 sind der Übersicht halber in der Produktionsstätte 18 im Unterschied zu der Lüftungsanlage 2 nur mit ihren für die Beschreibung der beanspruchten Erfindung zum Verständnis notwendigen Bestandteilen gezeigt. Die Förderaggregate 23, 23.1, 23.2, 23.3 sind auf dem Dach des Gebäudes 19 angeordnet. Bei der Produktionsstätte 18 umfasst die Lüftungsanlage 22 mehrere Förderaggregate 23 bis 23.3, damit in unterschiedlichen Arbeitsbereichen für eine unterschiedliche und von den übrigen Arbeitsbereichen unabhängig steuerbares Arbeitsbereichsklima eingestellt werden kann. Somit befinden sich die Luftaustrittsrohre nebst Aerosolaustrittsrohren der einzelnen Förderaggregate 23 bis 23.3 in unterschiedlichen Arbeitsbereichen und auch in unterschiedlichen Produktionsetagen 20, 21. In dem Gebäude 19 sind die jeweiligen Luftaustrittsrohre zusammen mit den jeweiligen Aerosolaustrittsrohren mit den Bezugszeichen 24 bis 24.4 kenntlich gemacht. Während sich die Luft- und Aerosolaustrittsrohre 24, die von dem Förderaggregat 23 beaufschlagt sind, sich in der oberen der beiden Produktionsetagen 20, 21 befindet, sind die übrigen Luft- und Aerosolaustrittsrohre 24.1 in der unteren Produktionsetage 20 angeordnet. Die Luft- und Aerosolaustrittsrohre 24 bis 24.4, befinden sich in jeweils unterschiedlichen Arbeitsbereichen des Gebäudes 19 in einer Höhe, dass durch diese die Produktion und der Flurverkehr nicht gestört sind.

Von dem Förderaggregat 23 sind gleichermaßen die Luft- und Aerosolaustrittsrohre 24 aus der oberen Produktionsetage 20 und die Luft- und Aerosolaustrittsrohre 24.1 aus einem Arbeitsbereich in der unteren Produktionsetage 20 beaufschlagt. Dieses erfolgt vor dem Hintergrund, dass bei dem dargestellten Ausführungsbeispiel diese beiden Arbeitsbereiche eine gleiche Klimatisierung und einen gleichen Arbeitsbereichdruck aufweisen sollen. Der den Luft- und Aerosolaustrittsrohren 24 zugeordnete Arbeitsbereich dient einer Backvorbereitung. In diesem Arbeitsbereich werden Teiglinge erstellt und zum Aufgehen gelagert, bevor diese in einen als Durchlaufofen konzipierten Backofen 25 eingebracht werden. Auch der Backofen 25 befindet sich in der oberen Produktionsetage 21, benachbart zu dem eben beschriebenen Arbeitsbereich. Die die beiden Produktionsetagen 20, 21 trennende Geschossdecke 26 ist in einem Teilbereich zur Ausbildung eines Luftraumes durchbrochen. Diese Durchbrechung 27 dient zum Bereitstellen einer Luftströmungswegsamkeit von der unteren Produktionsetage 20 in die obere Produktionsetage 21. Ein Fahrstuhlschacht 28, dessen Fahrstuhl 29 sich in der Darstellung der Figur 2 in der unteren Produktionsetage 20 befindet, dient bei diesem Ausführungsbeispiel als Sammelschacht, um die erzeugten Luftströmungen zu sammeln und als Abluft aus dem Dach 30 des Fahrstuhlschachtes 28 auszulassen. Mehrere Abluftklappen 31, 31.1 sind hinsichtlich ihrer Öffnungsweite ebenso einstellbar wie die Abluftklappe 9 der Lüftungsanlage 1. In Abhängigkeit von der Einstellung der Öffnungsweite der Abluftklappen 31, 31.1 werden die in dem Gebäude 19 generierten Luftströmungen beeinflusst.

Nicht dargestellt ist in Figur 2 eine Wärmerückgewinnung aus dem Abluftstrom, welche Wärme genutzt wird, um bedarfweise die zugeführte Zuluft zu erwärmen und/oder Wärme benötigenden Produktionsschritten zugeführt wird. Diesbezüglich kann eine Rückleitung erfolgen, wie dieses prinzipiell zu der Lüftungsanlage 2 der Figur 1 beschrieben ist.

Die in der unteren Produktionsetage 20 befindlichen Luft- und Aerosolaustrittsrohre 24.1, 24.2, 24.3, 24.4 erzeugen jeweils eine Luftströmung nach den zuvor beschriebenen Prinzipien. In Figur 2 sind die sich bei einem Betrieb der Lüftungsanlage 2 einstellenden Luftströmungen schematisiert durch gestrichelte Pfeile dargestellt. Die den Luft- und Aerosolaustrittsrohren 24.1 bis 24.4 jeweils zugeordneten Arbeitsbereiche sind durch Wände 32 voneinander getrennt. Bei dem dargestellten Ausführungsbeispiel erstrecken sich die Wände 32 nicht bis an die Geschossdecke 26. Der verbleibende Zwischenraum wird als Strömungsdurchbrechung genutzt, damit sich von jedem der zusammengefassten Luft- und Aerosolaustrittsrohre 24.1 bis 24.4 eine Luftströmung zu dem als Abluftsammelschacht genutzten Fahrstuhlschacht 28 bei einem Betrieb der Lüftungsanlage 2 ausbildet. Bei dem dargestellten Ausführungsbeispiel dienen die Arbeitsbereiche der Luft- und Aerosolaustrittsrohre 24.1, 24.2 einer Arbeitsvorbereitung. Der Arbeitsbereich mit dem Luft- und Aerosolaustrittsrohr 24.3 ist als Konditorei ausgeführt. Auf der anderen Seite des Fahrstuhlschachtes 28 befindet sich ein Arbeitsbereich, in dem die Luft- und Aerosolaustrittsrohre 24.4 angeordnet sind. Dieser Arbeitsbereich ist als Spül- und Reinigungsbereich eingerichtet. In diesem werden Transportgebinde, typischerweise Kunststoffkästen nach ihrer Benutzung gespült, um sodann wieder in dem Produktionsprozess verwendet werden zu können.

Bei einem Betrieb des Ofens 25 setzt dieser eine nicht unerhebliche Menge an Prozesswärme frei. Diese ist bestrebt aufzusteigen, und zwar in den Fahrstuhlschacht 28 hinein. Infolge der einsetzenden Konvektion entsteht in den kühleren Bereichen, und zwar auch in der unteren Produktionsetage 20 sowie in dem Arbeitsbereich, in den die Luft- und Aerosolaustrittsrohre 24 münden, ein gewisser Sog. Infolgedessen fungiert der Ofen 25 bei Betrieb auch als Luftströmungsmotor. Bei der Produktionsstätte 18 handelt es sich um eine solche, die prinzipiell 24 Stunden am Tag arbeitet. Daher befindet sich der als Luftströmungsmotor eingesetzte Ofen 25 ununterbrochen oder quasi ununterbrochen in Betrieb.

Die sich in dem Gebäude 19 einstellende Luftströmungen, ausgehend von jedem Luftaustrittsrohr, erstrecken sich, wie aus Figur 2 ersichtlich, zunächst vornehmlich in horizontaler Richtung in Richtung zu dem als Sammelschacht dienenden Fahrstuhlschacht 28 und sodann in vertikaler Richtung nach oben zu den Abluftklappen 31, 31.1.

Durch jedes Förderaggregat 23 bis 23.3 können die von diesem jeweils beaufschlagten Luft- und Aerosolaustrittsrohre unabhängig angesteuert werden. Dieses ermöglicht die Einstellung eines unterschiedlich starken Überdruckes im Bereich der Luftaustrittsrohre in jedem Arbeitsbereich, der durch ein Förderaggregat 23 bis 23.3 beaufschlagt ist, sowie eine Einstellung einer unabhängigen Arbeitsbereichluftfeuchte ebenso wie eine Arbeitsbereich unterschiedliche Umgebungsdruckeinstellung.

Es versteht sich, dass für die beschriebene Betriebsweise der Lüftungsanlagen 22 das Gebäude 19 nach Art eines Niedrigenergiehauses nach außen hin strömungstechnisch hinreichend dicht ist.

Die Zuführung der Arbeitsbereichluftfeuchte in Form von Aerosol ermöglicht es, dieses als Träger zum Zuführen von biologischen Stoffen in einem Arbeitsbereich zu nutzen. Dieses wird bei dem dargestellten Ausführungsbeispiel über das Förderaggregat 23.3 zum Ausbringen von biologisch aktiviertem Aerosol aus dem Aerosolaustrittsrohr der Luft- und Aerosolaustrittsrohre 24.4 genutzt. Diese Luft- und Aerosolaustrittsrohre 24.4 befinden sich im Arbeitsbereich "Spülen". Aufgrund der hohen Luftfeuchte besteht grundsätzlich die Gefahr einer Versottung. In zeitlichen Abständen wird das diesem Arbeitsbereich zugeführte Aerosol mit einem Fungizid beaufschlagt, und zwar typischerweise dann, wenn der Spülprozess ruht. Aus diesem Grunde ist der Spülarbeitsbereich auch von dem als Abluftstromsammler dienenden Fahrstuhlschacht 28 von den Lebensmittel ver- und bearbeitenden Arbeitsbereichen getrennt.

Die Erfindung ist anhand von Ausführungsbeispielen beschrieben worden.

Ohne den Umfang der geltenden Ansprüche zu verlassen, ergeben sich für einen Fachmann zahlreiche weitere Ausgestaltungen, die Erfindung verwirklichen zu können.

### Bezugszeichenliste

- 1: Gebäude
- 2: Lüftungsanlage
- 3: Förderaggregat
- 4: Dach
- 5: Gehäuse
- 6: Zuluftleitung
- 7: Luftaustrittskanal
- 8: Abluftöffnung
- 9: Abluftklappe
- 10: Innenraum
- 11: Abluftstutzen
- 12: Öffnung
- 13: Aerosolzuleitung
- 14: Aerosolaustrittsrohr
- 15, 15.1: Stütze
- 16: Wanne
- 17: Ablaufrohrstück
- 18: Produktionsstätte
- 19: Gebäude
- 20: Produktionsetage
- 21: Produktionsetage
- 22: Lüftungsanlage
- 23, 23.1, 23.2, 23.3: Förderaggregat
- 24, 24.1, 24.2, 24.3, 24.4: Luft- und Aerosolaustrittsrohre
- 25: Ofen
- 26: Geschossdecke
- 27: Durchbrechung
- 28: Fahrstuhlschacht
- 29: Fahrstuhl
- 30: Dach
- 31, 31.1: Abluftklappe
- 32: Wand

## Patentansprüche

1. Anordnung umfassend einen Innenraum (10) und eine Lüftungsanlage (2), welche Lüftungsanlage (2) ein Förderaggregat (3) zum Fördern eines Luftstroms umfasst, welches Förderaggregat (3) zumindest einen in dem Innenraum (10) angeordneten Luftaustrittskanal (7) mit einem Luftstrom beaufschlagt, wobei Teil der Lüftungsanlage (2) eine Befeuchtungseinrichtung zum Zuführen von Luftfeuchte in den Innenraum (10) ist, welche Befeuchtungseinrichtung einen Ultraschallvernebler zum Erzeugen eines Aerosols aus einer Flüssigkeit mit Flüssigkeitströpfchen in einer Größe, damit diese von der sich in dem Innenraum (10) einstellenden Luftströmung mitgeführt werden können, eine Aerosolzuleitung (13) zum Zuführen von Aerosol an zumindest einen Aerosolaustrittskanal sowie zumindest einen Aerosolaustrittskanal umfasst, der in dem Innenraum (10) dergestalt angeordnet ist, damit das austretende Aerosol von der Luftströmung erfassbar ist, **dadurch gekennzeichnet, dass** das Förderaggregat (3) zur Förderung eines Zuluftstroms in den Innenraum (10) vorgesehen ist und dieses Förderaggregat (3) auch den Ultraschallvernebler umfasst und dass der Innenraum (10) zumindest eine hinsichtlich ihrer Öffnungsweite einstellbare Abluftöffnung (8) aufweist, durch die Abluft aus dem Innenraum (10) austreten kann, wobei der zumindest eine Luftaustrittskanal (7) mit Abstand zu der zumindest einen Abluftöffnung (8) angeordnet ist, so dass sich bei einem Betrieb der Lüftungsanlage (2) in dem Innenraum (10) eine Luftströmung von dem zumindest einen Luftaustrittskanal (7) zu der zumindest einen Abluftöffnung (8) einstellt, wobei das austretende Aerosol von der Luftströmung erfasst wird und der zumindest eine Aerosolaustrittskanal als Aerosolaustrittsrohr (14) ausgeführt ist und jedes Aerosolaustrittsrohr (14) in Bezug auf seine Erstreckung parallel und in funktionaler Nachbarschaft zu einem Luftaustrittskanal (7) angeordnet ist.

2. Anordnung nach Anspruch 1, **dadurch gekennzeichnet, dass** der zumindest eine Luftaustrittskanal (7) als Textilrohr ausgeführt ist.

3. Anordnung nach Anspruch 1 oder 2, **dadurch gekennzeichnet, dass** das zumindest eine Luftaustrittskanal (7) in Bezug auf die Höhe des Innenraums (10) auf einem tieferen Niveau angeordnet ist als die wenigstens eine Abluftöffnung (8).

4. Anordnung nach einem der Ansprüche 1 bis 3, **dadurch gekennzeichnet, dass** das Förderaggregat (3) durch eine Decke (4) von dem Innenraum (10) getrennt ist.

5. Anordnung nach einem der Ansprüche 1 bis 4, **dadurch gekennzeichnet, dass** der Innenraum eine Produktionsstätte (18) ist, welche Produktionsstätte (18) ein geschlossenes Gebäude (19) mit vorzugsweise mehreren Arbeitsbereichen umfasst.

6. Anordnung nach Anspruch 5, **dadurch gekennzeichnet, dass** das Gebäude (19) der Produktionsstätte (18) durch Vorsehen von zumindest zwei Produktionsebenen (20, 21) mehrgeschossig ist, wobei die Geschosse (20, 21) durch wenigstens einen Strömungsdurchgang (27) zum Durchlassen der durch die Lüftungsanlage (22) erzeugten Luftströmung miteinander verbunden sind, dass ein oder mehrere Arbeitsbereiche in der unteren Produktionsebene (20) und ein oder mehrere weitere Arbeitsbereiche in der darüber befindlichen Produktionsebene vorgesehen sind und dass der Luftaustrittskanal in der unteren Produktionsebene (20) angeordnet ist.

7. Anordnung nach Anspruch 5, **dadurch gekennzeichnet, dass** zumindest eine Produktionsebene (20), in denen Luftaustrittskanäle angeordnet sind, in durch Wände (32) begrenzte Arbeitsbereiche gegliedert ist und in zumindest zwei Arbeitsbereichen jeweils wenigstens ein unabhängig von dem oder den in anderen Arbeitsbereichen angeordneten Luftaustrittskanälen mit einem Zuluftstrom beaufschlagbarer Luftaustrittskanal mit einem diesem zugeordneten Arosolaustrittsrohr angeordnet ist.

8. Anordnung nach Anspruch 6 oder 7, **dadurch gekennzeichnet, dass** die Produktionsebenen (20, 21) durch wenigstens einen Strömungsdurchgang (27) zwischen den Geschossen zum Durchlassen der durch die Lüftungsanlage (22) erzeugten Luftströmung miteinander verbunden sind.

9. Anordnung nach einem der Ansprüche 6 bis 8, **dadurch gekennzeichnet, dass** in wenigstens einem Arbeitsbereich eine Prozesswärme abgebende Produktionsmaschine, etwa ein Ofen (25), angeordnet ist und sich dieser Arbeitsbereich in einer Produktionsebene (21) oberhalb derjenigen Produktionsebene (20) befindet, in der Luftaustrittskanäle angeordnet sind.

10. Anordnung nach Anspruch 9, **dadurch gekennzeichnet, dass** die Prozesswärme abgebende Produktionsmaschine (25) innerhalb der Produktionsebene (21) dergestalt positioniert ist, damit die durch die Lüftungsanlage (22) erzeugte Luftströmung zumindest teilweise an dieser vorbei strömt.

11. Verfahren zum Klimatisieren eines Innenraumes unter Verwendung wenigstens einer Lüftungsanlage nach einem der Ansprüche 1 - 10, **dadurch gekennzeichnet, dass** durch wenigstens einen Luftaustrittskanal ein Zuluftstrom in das Gebäude (19) mit einem dem an dieser Stelle ohne Zuluftstrom herrschenden Druck höheren Druck eingebracht wird, welcher Zuluftstrom durch das Gebäude (19) oder einen Teil desselben zu wenigstens einer Abluftöffnung (31, 31.1) geleitet wird, und dass dem Zuluftstrom nach Austritt aus einem Luftaustrittskanal ein Aerosol mit Flüssigkeitstropfen in einer Größe beigemengt wird, damit diese von der sich in dem Gebäude (19) einstellenden Luftströmung mitgeführt werden.

12. Verfahren nach Anspruch 11, **dadurch gekennzeichnet, dass** der zu klimatisierende Innenraum zumindest zwei Ebenen (20, 21) aufweist und die sich durch einen Betrieb der Lüftungsanlage (22) einstellende Luftströmung zumindest teilweise das Gebäude (19) in vertikaler Richtung durchströmt.

13. Verfahren nach Anspruch 12, **dadurch gekennzeichnet, dass** die Luftstrombewegung in dem Gebäude (19) zumindest in einem ihrer vertikal strömenden Abschnitte durch ein oder mehrere Prozesswärme abgegebene Produktionsmaschinen (25) unterstützt oder erst ermöglicht wird.

14. Verfahren nach einem der Ansprüche 11 bis 13, **dadurch gekennzeichnet, dass** die Zuluft und das Aerosol in einer Produktionsebene (20) in mehreren Arbeitsbereichen, die durch Wände voneinander getrennt sind, zugeführt wird, wobei eine Zuführung der Zuluft und/oder des Aerosols in einzelnen Arbeitsbereichen unabhängig von der Zuluft- und/oder Aerosolzuführung in anderen Arbeitsbereichen eingestellt wird.

15. Verfahren nach einem der Ansprüche 11 bis 14, **dadurch gekennzeichnet, dass** das zuzuführende Aerosol mit einem biologischen Wirkstoff versetzt wird.

## Claims

1. Arrangement comprising an interior (10) and a ventilation system (2), said ventilation system (2) comprising a delivery device (3) for delivering an air flow, said delivery device (3) subjecting at least one air outlet channel (7) arranged in the interior (10) to an air flow, wherein part of the ventilation system (2) comprises a humidifying device to introduce air humidity into the interior (10), said humidifying device comprising an ultrasonic nebuliser for producing an aerosol from a liquid with liquid droplets in a size such that they can be conveyed together with the airflow which is to be established in the interior (10), an aerosol delivery line (13) for delivering aerosol to at least one aerosol outlet channel, and at least one aerosol outlet channel, which is arranged in the interior (10) in such a way that the emerging aerosol can be taken up by the air flow, **characterised in that** the delivery device (3) is provided such as to deliver an air inflow into the interior (10), and this delivery device (3) also comprises the ultrasonic nebuliser, and that the interior (10) also exhibits at least one waste air opening (8) which is adjustable in respect of its opening width, through which the waste air from the interior (10) can emerge, wherein the at least one air outlet channel (7) is arranged at a distance interval from the at least one waste air opening (8), such that, during the operation of the ventilation system (2), in the interior (10) an air flow is established from the at least one air outlet channel (7) to the at least one waste air opening (8), wherein the emerging aerosol is taken up by the air flow, and the at least one aerosol outlet channel is configured as an aerosol outlet tube (14), and each aerosol outlet tube (14) is arranged in respect of its extension parallel to and in functional proximity to an air outlet channel (7).

2. Arrangement according to claim 1, **characterised in that** the at least one air outlet channel (7) is configured as a textile tube.

3. Arrangement according to claim 1 or 2, **characterised in that** the at least one air outlet channel (7) is arranged at a lower level, in relation to the height of the interior (10), than the at least one waste air opening (8).

4. Arrangement according to any one of claims 1 to 3, **characterised in that** the delivery device (3) is separated from the interior (10) by a cover (4).

5. Arrangement according to any one of claims 1 to 4, **characterised in that** the interior is a production facility (18), this production facility (18) comprising a closed building (19) preferably with a plurality of working areas.

6. Arrangement according to claim 5, **characterised in that** the building (19) of the production facility (18) is multi-storey by the provision of at least two production levels (20, 21), wherein the storeys (20, 21) are connected to one another by at least one flow passage (27) for allowing through the air flow produced by the ventilation system (22), that one or more working areas are provided on the lower production level (20), and one or more further working areas are provided on the production level above this, and that the air outlet channel is arranged on the lower production level (20).

7. Arrangement according to claim 5, **characterised in that** at least one production level (20), in which air outlet channels are arranged, is divided into working areas delimited by walls (32), and arranged in at least two working areas in each case is at least one air outlet channel, which can be subjected to an air inflow independently of the air outlet channels in other working areas, with an aerosol outlet pipe allocated to this.

8. Arrangement according to claim 6 or 7, **characterised in that** the production levels (20, 21) are connected by at least one flow passage (27) between the storeys, to allow through the air flow produced by the ventilation system (22).

9. Arrangement according to any one of claims 6 to 8, **characterised in that** in at least working area a production machine giving off process heat is arranged, such as a furnace (25), and this working area is located on a production level (21) above that production level (20) on which the air outlet channels are arranged.

10. Arrangement according to claim 9, **characterised in that** the production machine (25) giving off process heat is positioned inside the production level (21) in such a way that the air flow produced by the ventilation system (22) at least in part flows past it.

11. Method for air-conditioning of an interior, making use of at least one ventilation system according to any one of claims 1-10, **characterised in that** an inlet air flow is introduced into the building (19) through at least one air outlet channel, at a higher pressure than the pressure which prevails at this point without inlet air flow, this inlet air flow being conveyed through the building (19) or a part thereof, to at least one waste air opening (31, 31.1), and that, after emerging from an air outlet channel, an aerosol is mixed into the air inlet flow, with liquid droplets of a size such that they are conveyed by the air flow produced in the building (19).

12. Method according to claim 11, **characterised in that** the interior which is to be air-conditioned comprises at least two levels (20, 21), and the air flow which is produced by the operation of the ventilation system (22) flows at least in part through the building (19) in a vertical direction.

13. Method according to claim 12, **characterised in that** the air flow movement in the building (19) is supported or first made possible, at least in one of its vertical flowing sections, by one or more production machines (25) giving off process heat.

14. Method according to any one of claims 11 to 13, **characterised in that** the incoming air and the aerosol are introduced on a production level (20) in several working areas which are separated from one another by walls, wherein an inflow of the incoming air and/or of the aerosol is adjusted in individual working areas independently of the introduction of incoming air and/or aerosol in other working areas.

15. Method according to any one of claims 11 to 14, **characterised in that** the aerosol being introduced has a biological active substance added to it.

## Revendications

1. Arrangement comprenant une pièce (10) et un système de ventilation (2), lequel système de ventilation (2) comprend un groupe de transport (3) destiné à transporter un flux d'air, lequel groupe de transport (3) envoie un flux d'air vers au moins un canal de sortie d'air (7) disposé dans la pièce (10), un humidificateur faisant partie du système de ventilation (2) destiné à acheminer de l'air humide dans la pièce (10), lequel humidificateur comprend un brumisateur à ultrasons destiné à générer un aérosol à partir d'un liquide avec des gouttelettes de liquide dont la taille permet qu'elles soient emportées par la circulation d'air s'instaurant dans la pièce (10), une conduite d'arrivée d'aérosol (13) afin d'acheminer l'aérosol vers au moins un canal de sortie d'aérosol, et au moins un canal de sortie d'aérosol, disposé de telle manière dans la pièce (10) que l'aérosol qui en sort peut être emporté par la circulation d'air, **caractérisé en ce que** le groupe de transport (3) est prévu pour transporter un flux d'air d'arrivée dans la pièce (10) et que ce groupe de transport (3) comprend également le brumisateur à ultrasons et que la pièce (10) présente au moins une ouverture d'échappement d'air (8) réglable au niveau de sa largeur d'ouverture, laquelle permet l'échappement d'air de la pièce (10), au moins un canal de sortie d'air (7) étant disposé à distance de l'au moins une ouverture d'échappement d'air (8), de sorte qu'il s'instaure dans la pièce (10), quand le système de ventilation (2) fonctionne, une circulation d'air depuis l'au moins un canal de sortie d'air (7) vers l'au moins une ouverture d'échappement d'air (8), l'aérosol émis étant entraîné par la circulation d'air et l'au moins un canal de sortie d'aérosol étant conformé en tuyau de sortie d'aérosol (14) et chaque tuyau de sortie d'aérosol (14) étant disposé en ce qui concerne son étendue, parallèlement et au voisinage fonctionnel d'un canal de sortie d'air (7).

2. Arrangement selon la revendication 1, **caractérisé en ce que** l'au moins un canal de sortie d'air (7) est conformé en tuyau textile.

3. Arrangement selon la revendication 1 ou 2, **caractérisé en ce que** l'au moins un canal de sortie d'air (7) est disposé, par rapport à la hauteur de la pièce (10) à un niveau inférieur à l'au moins une ouverture d'échappement d'air (8).

4. Arrangement selon l'une des revendications 1 à 3, **caractérisé en ce que** le groupe de transport (3) est séparé de la pièce (10) par un plafond (4).

5. Arrangement selon l'une des revendications 1 à 4, **caractérisé en ce que** la pièce est un site de production (18), lequel site de production (18) comprend un bâtiment (19) fermé avec de préférence plusieurs zones de travail.

6. Arrangement selon la revendication 5, **caractérisé en ce que** le bâtiment (19) du site de production (18) comporte plusieurs étages en prévoyant au moins deux niveaux de production (20, 21), lesquels étages (20, 21) étant reliés par au moins un passage (27) de circulation afin de laisser passer la circulation d'air générée par le système de ventilation (22), **en ce qu'**il est prévu une ou plusieurs zones de travail au niveau de production (20) inférieur et une ou plusieurs zones de travail au niveau de production en surplomb et **en ce que** le canal de sortie d'air est disposé dans le niveau de production (20) inférieur.

7. Arrangement selon la revendication 5, **caractérisé en ce qu'**au moins un niveau de production (20), dans lequel sont disposés des canaux de sortie d'air, est compartimenté en zones de travail délimitées par des murs (32) et que dans au moins deux zones de travail est disposé respectivement au moins un canal de sortie d'air qui peut être approvisionné par un flux d'air d'arrivée, qui est indépendant du ou des canaux de sortie d'air disposés dans les autres zones de travail, et qui comprend un tuyau de sortie d'aérosol qui lui est affecté.

8. Arrangement selon la revendication 6 ou 7, **caractérisé en ce que** les niveaux de production (20, 21) sont reliés l'un à l'autre par au moins un passage (27) de circulation entre les étages destinés à laisser passer la circulation flux d'air générée par le système de ventilation (22).

9. Arrangement selon l'une des revendications 6 à 8, **caractérisé en ce qu'**une machine de production dégageant de la chaleur de process, par exemple un four (25), est disposée dans au moins une zone de travail et que cette zone de travail se trouve à un niveau de production (21) au-dessus du niveau de production (20) où sont disposés les canaux de sortie d'air.

10. Arrangement selon la revendication 9, **caractérisé en ce que** la machine de production (25) dégageant de la chaleur de process est positionnée au sein du niveau de production (21) de telle manière que la circulation d'air généré par le système de ventilation (22) passe au moins partiellement à côté de celle-ci.

11. Procédé pour climatiser une pièce en utilisant au moins un système de ventilation selon l'une des revendications 1 à 10, **caractérisé en ce qu'**un flux d'air d'arrivée est introduit à travers au moins un canal de sortie d'air dans le bâtiment (19) à une pression supérieure à la pression qui règne à cet endroit sans le flux d'air d'arrivée, lequel flux d'air d'arrivée est guidé à travers le bâtiment (19) ou une partie de celui-ci vers au moins une ouverture d'échappement d'air (31, 31.1) et **en ce qu'**il est mélangé au flux d'air d'arrivée après sa sortie d'un canal de sortie d'air, un aérosol comportant des gouttes de liquide dont la taille est telle que celles-ci peuvent être entraînées par la circulation d'air qui s'instaure dans le bâtiment (19).

12. Procédé selon la revendication 11, **caractérisé en ce que** la pièce à climatiser présente au moins deux niveaux (20, 21) et que la circulation d'air qui s'instaure du fait du fonctionnement d'un système de ventilation (22) traverse au moins partiellement le bâtiment (19) dans le sens vertical.

13. Procédé selon la revendication 12, **caractérisé en ce que** le mouvement du flux d'air dans le bâtiment (19) dans au moins un de ses tronçons de circulation verticale est soutenu voire rendu possible par une ou plusieurs machines de production (25) dégageant une chaleur de process.

14. Procédé selon l'une des revendications 11 à 13, **caractérisé en ce que** l'air d'arrivée et l'aérosol sont acheminés vers un niveau de production (20) compartimenté en plusieurs zones de travail, séparées les unes des autres par des murs, l'acheminement de l'air d'arrivée et/ou de l'aérosol dans les différentes zones de travail étant réglé indépendamment de l'arrivée d'air et/ou de l'acheminement d'aérosol dans les autres zones de travail.

15. Procédé selon l'une des revendications 11 à 14, **caractérisé en ce que** l'aérosol à acheminer est modifié par une substance active biologique.
